# EUROPÄISCHE PATENTSCHRIFT

(11) **EP 2 593 094 B1**
(45) Veröffentlichungstag und Bekanntmachung des Hinweises auf die Patenterteilung: **06.12.2017**
(21) Anmeldenummer: 11733637.0
(22) Anmeldetag: 07.07.2011
(51) Int. Cl.: A61K 31/137, A61K 9/00

(54) **WÄSSRIGE ZUSAMMENSETZUNG ENTHALTEND BROMHEXIN**
AQUEOUS COMPOSITION COMPRISING BROMHEXINE
COMPOSITION AQUEUSE CONTENANT DE LA BROMHEXINE

(30) Priorität: 12.07.2010 EP 10169236
(43) Veröffentlichungstag der Anmeldung: 22.05.2013
(73) Patentinhaber: Sanofi-Aventis Deutschland GmbH, 65929 Frankfurt am Main (DE)
(72) Erfinder: SCHEURING, Uwe, 55216 Ingelheim am Rhein (DE); PLOHMANN, Bernd, 55216 Ingelheim am Rhein (DE); ZAMPONI, Annette, 55216 Ingelheim am Rhein (DE)
(74) Vertreter: Catillon, Marie
(86) Internationale Anmeldenummer: PCT/EP2011/061527
(87) Internationale Veröffentlichungsnummer: WO 2012/007352

(56) Entgegenhaltungen:
- EP-A1- 0 896 815
- EP-A1- 1 121 940
- WO-A1-03/030877
- DATABASE WPI Week 200145 Thomson Scientific, London, GB; AN 2001-420810 XP002609724, & JP 2001 106639 A (TAISHO PHARM CO LTD) 17. April 2001 (2001-04-17)
- DATABASE WPI Week 200981 Thomson Scientific, London, GB; AN 2009-R19749 XP002609777, & KR 2009 115 359 A (ELT SCI CORP) 5. November 2009 (2009-11-05)
- DATABASE EPODOC [Online] EUROPEAN PATENT OFFICE, THE HAGUE, NL; 22. August 2007 (2007-08-22), "Composition and great volume injection containing bromhexine salt and the injection preparing process", XP002609725, Database accession no. CN101019826 A & CN 101 019 826 A (ZHANG SONG [CN] SONG ZHANG [CN]) 22. August 2007 (2007-08-22)

## Beschreibung

Es ist bekannt, dass Bromhexin ein synthetisches Derivat des pflanzlichen Wirkstoffes Vasicin ist. Es wirkt sekretolytisch und sekretomotorisch im Bereich des Bronchialtraktes. In klinischen Studien wurde gezeigt, dass es den Husten lindert und das Abhusten erleichtert. Durch die Verminderung der Viskosität und die Aktivierung des Flimmerepithels wird der Abtransport des Schleims gefördert. Demzufolge wird Bromhexin zur sekretolorischen Therapie bei akuten und chronischen bronchopulmonalen Erkrankungen, die mit einer Störung von Schleimhautbildung und -transport einhergehen, verwendet.

Es ist ferner bekannt, dass Bromhexin Hydrochlorid bei Lagerung einem langsamen Zersetzungsprozess unterliegen. Zersetzungsprodukte sind gemäß der Europäischen Monographie zu Bromhexin Hydrochlorid (Ph Eur monograph 0706) insbesondere (A) (2-Amino-3,5-dibromphenyl)methanol, (B) 2-Amino-3,5-dibrom-benzaldehyd, (C) N-(2-Aminobenzyl)-N-methylcyclohexanamin, (D) N-(2-Amino-5-brombenzyl)-N-methylcyclohexanamin und (E) (3RS)-6,8-Dibromo-3-cyclohexyl-3-methyl-1,2,3,4-tetrahydrochinazolin-3-ium.

Zur Stabilisierung bromhexinhaltiger Zusammensetzung lehrt die JP 10101581 die Verwendung von Reduktionsmitteln und/oder Chelatkomplexbildnern.

Die JP 200281562 und JP 2007119453 lehren die Verwendung von Zuckeralkoholen zur Stabilisierung bromhexinhaltiger Zusammensetzung. Die JP 63313725 lehrt Maltitol als besonders geeigneten Stabilisator. Die JP 10036292 und JP10306038 lehren als weiteren Vorteil der Zuckeralkohole die Maskierung des bitteren Eigengeschmacks von Bromhexin.

Eine besonders stabile wässrige Zusammensetzung enthaltend Bromhexin wird unter dem Namen Bisolvon® Hustensaft (Lösung zum Einnehmen) vermarktet. Diese Zusammensetzung enthält Bromhexin Hydrochlorid (8 mg/5 ml), Maltitol-Sirup, Benzoesäure, Levomenthol, Sucralose, Aromastoffe und gereinigtes Wasser.

Aufgabe der vorliegenden Erfindung war die Bereitstellung einer wässrigen Lösung von Bromhexin, die sich durch eine besonders hohe Stabilität auszeichnet, d.h. durch eine besonders niedrige Zersetzungsrate von Bromhexin. Hierdurch sollte insbesondere eine Eignung der Zusammensetzung zur Abfüllung in Folienverpackungen erzielt werden. Weiterhin sollte sich die erfindungsgemäße Zusammensetzung nach Möglichkeit durch eine ausreichende Maskierung des bitteren Geschmacks von Bromhexin auszeichnen. Weiterhin sollte sich die erfindungsgemäße Zusammensetzung nach Möglichkeit auch durch eine für das Mundgefühl geeignete Textur auszeichnen

Diese Aufgaben werden durch eine wässrige Zusammensetzung enthaltend Bromhexin, wobei die Zusammensetzung eine Menge an Zuckeralkoholen von weniger als 10 g bezogen auf 100 ml der Zusammensetzung aufweist, gelöst.

Genauer werden diese Aufgaben erfindungsgemäß durch eine wässrige Zusammensetzung, bestehend aus:
a) Bromhexin in einer Menge von 0.04 g bis 0.4 g,
b) Verdickungsmittel in einer Menge von 0.005 g bis 5 g,
c) Süssungsmittel in einer Menge von 0.01 g bis 10 g,
d) weiteren geeigneten Zusatzstoffen ausgewählt unter Konservierungsstoffen, Säureregulatoren, Entschäumern, Aromastoffen und Farbstoffen in einer Menge von 0 bis 10 g und
e) Wasser ad 100 ml,
wobei die Zusammensetzung eine Menge an Zuckeralkoholen von weniger als 10 g bezogen auf 100 ml der Zusammensetzung aufweist, gelöst.

Die erfindungsgemäßen Zusammensetzungen zeichnen sich überraschenderweise, trotz des geringen Anteils an zur Stabilisierung geeigneten Zuckeralkoholen, durch eine sehr geringe Zersetzungsrate von Bromhexin aus. Folglich kann auf die im Stand der Technik gelehrte Zugabe von Reduktionsmitteln und/oder Chelatkomplexbildnern verzichtet werden.

Die erfindungsgemäßen Zusammensetzungen eignen sich aufgrund der an sich bekannten Wirkung von Bromhexin in besonderer Weise als Hustensaft bzw. Hustensirup.

Demzufolge betrifft ein weiterer Gegenstand der vorliegenden Erfindung die Verwendung einer erfindungsgemäßen Zusammensetzung wie hierin definiert zur sekretolorischen Therapie bei akuten und chronischen bronchopulmonalen Erkrankungen.

Im Rahmen der vorliegenden Erfindung steht der Begriff "Bromhexin" für N-(2-Amino-3,5-dibrombenzyl)-N-methylcyclohexanamin Hydrochlorid.

Üblicherweise enthalten die erfindungsgemäßen Zusammensetzungen Bromhexin in einer Menge von 0.04 bis 0.4 g bezogen auf 100 ml der Zusammensetzung. Die angegebene Menge an Bromhexin bezieht sich jeweils auf die Menge des verwendeten Salzes. Bevorzugt enthalten die erfindungsgemäßen Zusammensetzungen Bromhexin in einer Menge von 0.08 bis 0.32 g, beispielsweise in einer Menge von 0.16 g, jeweils bezogen auf 100 ml der Zusammensetzung.

Eine spezielle Ausführungsform der vorliegenden Erfindung betrifft erfindungsgemäße Zusammensetzungen, die Bromhexin als alleinigen Wirkstoff enthalten, d.h. sogenannte Monopräparate.

Im Rahmen der vorliegenden Erfindung steht der Begriff "Zuckeralkohol" für Verbindungen die durch Reduktion eines Saccharids, insbesondere eines Mono- oder Disaccharids, erhältlich sind. Übliche Zuckeralkohole als Zusatzstoffe pharmazeutischer Zusammensetzungen sind beispielsweise Sorbit, Xylit, Maltit, Isomalt, Mannit, Threit, Erythrit und Arabit.

Die Menge an Zuckeralkoholen in den erfindungsgemäßen Zusammensetzungen beträgt weniger als 10 g bezogen auf 100 ml der Zusammensetzung. Bevorzugt beträgt die Menge an Zuckeralkoholen in den erfindungsgemäßen Zusammensetzungen weniger als 5 g und besonders bevorzugt weniger als 1 g, jeweils bezogen auf 100 ml der Zusammensetzung. Eine spezielle Ausführungsform der Erfindung betrifft Zusammensetzungen, die frei von Zuckeralkoholen sind.

Im Rahmen der vorliegenden Erfindung steht der Begriff "wässrige Zusammensetzungen" für flüssige Zusammensetzungen, deren Lösungsmittel zu wenigstens 50 Gew.-% aus Wasser besteht. Das übrige Lösungsmittel ist hierbei üblicherweise ausgewählt unter Alkoholen, wie Ethanol, Polyethylenglykol (Macrogol), Propylenglykol und Glycerol. Bevorzugt bezeichnet der Begriff "wässrige Zusammensetzungen" jedoch Zusammensetzungen, deren Lösungsmittel zu wenigstens 80 Gew.-% und besonders bevorzugt zu wenigstens 90 Gew.-% aus Wasser bestehen. Eine spezielle Ausführungsform betrifft erfindungsgemäße Zusammensetzung, deren Lösungsmittel ausschließlich aus Wasser besteht. Solche Zusammensetzungen sind alkoholfrei und somit grundsätzlich für die Verwendung bei Kindern geeignet.

Der Anteil des Lösungsmittels an der erfindungsgemäßen Zusammensetzung beträgt üblicherweise wenigstens 50 Gew.-%. Die erfindungsgemäßen Zusammensetzungen zeichnen sich bevorzugt durch einen Lösungsmittelgehalt von wenigstens 70 Gew.-% und besonders bevorzugt wenigstens 80 Gew.-% aus. Die erfindungsgemäßen Zusammensetzungen erlauben aufgrund des Verzichts auf Zuckeralkohole zur Stabilisierung auch einen Lösungsmittelgehalt von wenigstens 90 oder wenigstens 95 Gew.-%.

Üblicherweise enthalten die erfindungsgemäßen Zusammensetzungen einen geeigneten Verdicker. Hierdurch können die erfindungsgemäßen Zusammensetzungen in Abhängigkeit der gewünschten Darreichungsform auf die erforderliche Viskosität eingestellt werden. Folglich enthalten die erfindungsgemäßen Zusammensetzungen den Verdicker in einer Menge, die zur Einstellung der gewünschten Viskosität geeignet ist. Üblicherweise enthalten die erfindungsgemäßen Zusammensetzungen den Verdicker in einer Menge von 0.05 bis 5 g bezogen auf 100 ml der erfindungsgemäßen Zusammensetzung.

Geeignete Verdicker sind beispielsweise ausgewählt unter Hydroxypropylmethylcellulose (HPMC), Hydroxyethylcellulose (HEC), Hydroxypropylcellulose (HPC), Methylcellulose (MC), Carboxymethylcellulose (CMC), und Methylethylcellulose (MEC). Bevorzugt sind die Verdicker ausgewählt unter Hydroxypropylmethylcellulose (HPMC), Hydroxyethylcellulose (HEC) und Hydroxypropylcellulose (HPC). Insbesondere ist der erfindungsgemäß verwendete Verdicker Hydroxyethylcellulose.

Die erfindungsgemäßen Zusammensetzungen zeichnen sich üblicherweise durch eine Viskosität im Bereich von 50 mPas bis 30 Pas bei einer Temperatur von 20°C aus.

Eine spezielle Ausführungsform betrifft erfindungsgemäße Zusammensetzung, wobei die Zusammensetzung ein Hustensaft oder Hustensirup ist. Diese speziellen Darreichungsformen weisen üblicherweise eine Viskosität von wenigstens 100 mPas und insbesondere von wenigstens 120 mPas auf, jeweils bei einer Temperatur von 20°C. Die Menge an enthaltenem Verdicker liegt hier üblicherweise im Bereich von 0.1 bis 1 g bezogen auf 100 ml der erfindungsgemäßen Zusammensetzung.

Üblicherweise enthalten die erfindungsgemäßen Zusammensetzungen ein geeignetes Süssungsmittel. Bevorzugt werden erfindungsgemäß Süssungsmittel verwendet, die von Zuckeralkoholen verschieden sind, d.h. die erfindungsgemäßen Zusammensetzungen sind in dieser Ausführungsform frei von Zuckeralkoholen.

Geeignete Süssungsmittel sind beispielsweise ausgewählt unter Sucralose, Acesulfam, Aspartam, Cyclamat, Saccharin, Isomalt, Maltit, Xylit, Lactit, Erythrit, Alitame, Thaumatin und Neohesperidindihydrochalkon. Bevorzugt eignen sich Sucralose, Acesulfam, Aspartam, Cyclamat, Saccharin, Alitame, Thaumatin und Neohesperidindihydrochalkon. Besonders bevorzugt ist das Süssungsmittel Sucralose.

Die erfindungsgemäßen Zusammensetzungen enthalten das Süssungsmittel üblicherweise in einer Menge von 0.01 g bis 10 g bezogen auf 100 ml der Zusammensetzung. Bevorzugt enthalten die erfindungsgemäßen Zusammensetzungen das Süssungsmittel in einer Menge von 0.1 bis 1 g und besonders bevorzugt in einer Menge von 0.1 bis 0.5 g, jeweils bezogen auf 100 ml der erfindungsgemäßen Zusammensetzung.

Üblicherweise enthalten die erfindungsgemäßen Zusammensetzungen ein geeignetes Konservierungsmittel.

Aus Gründen der Klarheit bezieht sich der Begriff "Konservierungsstoff" wie hierin verwendet nicht auf Zuckeralkohole, die bekanntermaßen ebenfalls eine konservierende Wirkung aufweisen.

Geeignete Konservierungsstoffe sind beispielsweise Benzoesäure, Sorbinsäure, Schweflige Säure oder deren Salze. Insbesondere Benzoesäure hat sich für bromhexinhaltige Zusammensetzungen als geeigneter Konservierungsstoff bewährt.

Die erfindungsgemäßen Zusammensetzungen enthalten das Konservierungsmittel üblicherweise in einer Menge von 0.005 bis 0.5 g bezogen auf 100 ml der Zusammensetzung. Bevorzugt enthalten die erfindungsgemäßen Zusammensetzungen das Konservierungsmittel in einer Menge 0.01 bis 0.1 g, besonders bevorzugt von 0.02 bis 0.05 g, jeweils bezogen auf 100 ml der Zusammensetzung.

Üblicherweise weisen die erfindungsgemäßen Zusammensetzungen einen pH-Wert im Bereich von 2.0 bis 6.0 auf, bevorzugt von 2.5 bis 4.5. Besonders bevorzugt liegt der pH-Wert der erfindungsgemäßen Zusammensetzungen in einem Bereich von 3.0 bis 4.0.

Geeignete Säureregulatoren sind beispielsweise Apfelsäure, Fumarsäure, Milchsäure, Zitronensäure, Weinsäure, Orthophosphorsäure, Metaweinsäure, Adipinsäure oder Bernsteinsäure.

Wird eine Säure oder ihr Salz in den erfindungsgemäßen Zusammensetzungen als Konservierungsmittel verwendet, wird man üblicherweise auf die Verwendung eines zusätzlichen Säureregulators verzichten. In dieser speziellen Ausführungsform enthält die erfindungsgemäße Zusammensetzung daher üblicherweise keinen Säureregulator.

In einer weiteren bevorzugten Ausführungsform enthält die erfindungsgemäße Zusammensetzung keine Reduktionsmittel und/oder Chelatkomplexbildner, wie Weinsäure, EDTA oder Ähnliche, enthalten.

Zusammensetzung nach einem der vorhergehenden Ansprüche, bestehend aus:
a) Bromhexin in einer Menge von 0.04 g bis 0.4 g,
b) Verdickungsmittel in einer Menge von 0.005 g bis 5 g,
c) Süssungsmittel in einer Menge von 0.01 g bis 10 g,
d) weiteren geeigneten Zusatzstoffen ausgewählt unter Konservierungsstoffen, Säureregulatoren, Entschäumern, Aromastoffen und Farbstoffen in einer Menge von 0 bis 10 g und
e) Wasser ad 100 ml.

Bezüglich der bevorzugten Menge und Beschaffenheit der Komponenten dieser speziellen Ausführungsform gilt das zuvor Gesagte.

Geeignte Entschäumer, wie beispielsweise Simethicon, sind dem Fachmann bekannt Geeignete Aromastoffe und Farbstoffe sind dem Fachmann bekannt.

Die Herstellung der erfindungsgemäßen Zusammensetzungen erfolgt durch übliche Formulierungstechniken. Dabei ist es unkritisch ob die Bestandteile der erfindungsgemäßen Zusammensetzung gleichzeitig oder sukzessive miteinander vermischt werden. Ebenso ist es unerheblich in welcher Reihenfolge Die Bestandteile der erfindungsgemäßen Zusammensetzung können in reiner Form, in Form von Lösungen, oder in Form von Teilzusammensetzungen, die bereits mehrere Bestandteilen der erfindungsgemäßen Zusammensetzung enthalten, bereitgestellt werden.

Die erfindungsgemäßen Zusammensetzungen eignen sich aufgrund ihrer hohen Stabilität zur Abfüllung in allen üblichen Verpackungsformen. Insbesondere eignen sich die erfindungsgemäßen Zusammensetzungen neben der konventionellen Abfüllung in Glasbehältern aufgrund ihrer hohen Stabilität auch für die Abfüllung in chemisch inerten Folienverpackungen. Geeignete Folien sind kommerziell erhältlich. Insbesondere eignen sich für die Folienverpackung der erfindungsgemäßen Zusammensetzungen laminierte Folien, wie sie beispielsweise unter den Handelsnamen Aclar® oder Barex® kommerziell erhältlich sind.

Im Folgenden wird die Erfindung anhand nicht einschränkender Beispiele näher erläutert.

### Beispiele

### (A) Herstellungsbeispiel:

Zu 60 ml Wasser wurde unter Rühren bei 20 - 25°C Hydroxyethylcellulose gegeben. Das so erhaltene Gemisch wurde weitere 30 min. gerührt und anschließend Unter Rühren über einen Zeitraum von 60 min. auf ca. 85°C erwärmt. Im Anschluss wurde Benzoesäure (25.4 mg) zugegeben und weitere 10 min. gerührt. Im Anschluss wurden 35 ml Wasser zugegeben und die Lösung auf 60°C abgekühlt. Bromhexinhydrochlorid (160 mg) wurde zugegeben und 20 min. gerührt. Danach wurde die Lösung auf 50°C gekühlt, Sucralose (225 mg) zugegeben und 10 min. gerührt. Im Anschluss wurde auf Raumtemperatur abgekühlt und Aromastoffe (41 mg) zugegeben. Nach weiteren 30 min. unter Rühren erfolgte das Auffüllen auf ein Gesamtvolumen von 100ml durch die Zugabe von Wasser. Danach wurde die erhaltene Zusammensetzung 10 min. gerührt, filtriert und in Sachets auf der Stickpackmaschine bzw. in Braunglasflaschen abgefüllt.

Die so erhaltene erfindungsgemäße Zusammensetzung hat eine Viskosität von 135 mPas. Die Viskosität wurde bei einer Temperatur von 20 °C mittels Kugelfallviskosimeter nach der Methode der Europäischen Pharmacopoe bestimmt (European Pharmacopoeia, 6th edition, page 84, chapter 2.2.49).

### (B) Untersuchungen zur Stabilität

Zur Untersuchung der Stabilität wurde die unter (A) erhaltene Zusammensetzung sowie eine dem Bisolvon® Hustensaft (Vergleichsbeispiel) entsprechende Zusammensetzung jeweils in Folienverpackung (hergestellt aus Barex®-Folien der Fa. DanaPak) und in eine handelsübliche Braungasflasche abgefüllt. Die Stabilität der Zusammensetzung bei kontrollierten Lagerbedingungen (30 °C, 75 % r.h.) wurde anhand des in der Europäischen Monographie beschriebenen Zersetzungsproduktes E ((3RS)-6,8-Dibromo-3-cyclohexyl-3-methyl-1,2,3,4-tetrahydrochinazolin-3-ium) evaluiert. Hierzu wurde der Gehalt der Proben an Zersetzungsprodukt E durch HPLC und UV-Detektion bestimmt.

Die Ergebnisse dieser Untersuchung sind im Folgenden
zusammengefasst.

### a) Bisolvon® Hustensaft (Vergleichsbeispiel)

Zusammensetzung: Bromhexin Hydrochlorid (0.16 g/ 100 ml), Maltitol liquid (50 g), Benzoesäure (0.13 g/ 100 ml), Geschmackstoffe, Wasser.

| | | |
|---|---|---|
| Folienverpackung: | 12 Tage | 1 Monat, 30°C/75% r.h |
| Zersetzungsprodukt E: | 0.16% | 0.21% |
| Andere Zersetzungsprodukte: | nicht bestimmbar | < 0.05% |

| | | |
|---|---|---|
| Braunglassflasche: | 12 Tage | 1 Monat, 30°C/75% r.h |
| Zersetzungsprodukt E: | 0.16% | 0.21% |
| Andere Zersetzungsprodukte: | nicht bestimmbar | < 0.05% |

### b) Erfindungsgemäße Zusammensetzung

Zusammensetzung: gemäß Herstellungsbeispiel (A).

| | | |
|---|---|---|
| Folienverpackung: | 0 Tage | 1 Monat 30°C/75% r.h |
| Zersetzungsprodukt E: | <0.05% | 0.07% |
| Andere Zersetzungsprodukte: | nicht bestimmbar | 0.07% |

| | | |
|---|---|---|
| Braunglassflasche: | 0 Tage | 1 Monat 30°C/75% r.h |
| Zersetzungsprodukt E: | < 0.05% | < 0.05% |
| Andere Zersetzungsprodukte: | 0.08% | nicht bestimmbar |

Die Versuchsergebnisse zeigen, dass sich die erfindungsgemäßen Zusammensetzungen gegenüber den handelsüblichen bromhexinhaltigen Zusammensetzungen durch eine besonders geringe Zersetzungsrate von Bromhexin auszeichnen.

## Patentansprüche

1. Wässrige Zusammensetzung, bestehend aus:
a) Bromhexin in einer Menge von 0.04 g bis 0.4 g,
b) Verdickungsmittel in einer Menge von 0.005 g bis 5 g,
c) Süssungsmittel in einer Menge von 0.01 g bis 10 g,
d) weiteren geeigneten Zusatzstoffen ausgewählt unter Konservierungsstoffen, Säureregulatoren, Entschäumern, Aromastoffen und Farbstoffen in einer Menge von 0 bis 10 g und
e) Wasser ad 100 ml.
wobei die Zusammensetzung eine Menge an Zuckeralkoholen von weniger als 10 g bezogen auf 100 ml der Zusammensetzung aufweist.

2. Zusammensetzung nach Anspruch 1, wobei die Zusammensetzung eine Viskosität im Bereich von 50 mPas bis 30 Pas bei einer Temperatur von 20°C aufweist.

3. Zusammensetzung nach einem der vorhergehenden Ansprüche, enthaltend ein geeignetes Konservierungsmittel.

4. Zusammensetzung nach Anspruch 3, wobei das Konservierungsmittel in einer Menge von 0,01 g bis 0,5 g bezogen auf 100 ml der Zusammensetzung enthalten ist.

5. Zusammensetzung nach einem der vorhergehenden Ansprüche, wobei die Zusammensetzung einen pH-Wert im Bereich von 2.0 bis 6.0 aufweist.

6. Zusammensetzung nach einem der vorhergehenden Ansprüche, wobei die Zusammensetzung einen Gehalt an Wasser von wenigstens 70 Gew.-% aufweist.

7. Zusammensetzung nach einem der vorhergehenden Ansprüche, wobei die Zusammensetzung ein Hustensaft oder Hustensirup ist.

8. Verwendung einer Zusammensetzung nach einem der vorhergehenden Ansprüche zur Verwendung bei der sekretolorischen Therapie bei akuten und chronischen bronchopulmonalen Erkrankungen.

## Claims

1. Aqueous composition consisting of:
a) bromhexine in an amount of from 0.04 g to 0.4 g,
b) thickener in an amount of from 0.005 g to 5 g,
c) sweetener in an amount of from 0.01 g to 10 g,
d) further suitable additives selected from preservatives, acidity regulators, antifoams, flavourings and dyes in an amount of from 0 to 10 g and
e) water to 100 ml,
wherein the composition comprises an amount of sugar alcohols of less than 10 g, based on 100 ml of the composition.

2. Composition according to Claim 1, wherein the composition has a viscosity in the range of from 50 mPas to 30 Pas at a temperature of 20°C.

3. Composition according to either of the preceding claims, containing a suitable preservative.

4. Composition according to Claim 3, wherein the preservative is present in an amount of from 0.01 g to 0.5 g, based on 100 ml of the composition.

5. Composition according to any of the preceding claims, wherein the composition has a pH in the range of from 2.0 to 6.0.

6. Composition according to any of the preceding claims, wherein the composition has a content of water of at least 70% by weight.

7. Composition according to any of the preceding claims, wherein the composition is a cough mixture or cough syrup.

8. Use of a composition according to any of the preceding claims for use in the secretolory therapy in acute and chronic bronchopulmonary disorders.

## Revendications

1. Composition aqueuse, constituée par :
a) de la bromhexine en une quantité de 0,04 g à 0,4 g,
b) des épaississants en une quantité de 0,005 g à 5 g,
c) des édulcorants en une quantité de 0,01 g à 10 g,
d) d'autres additifs appropriés choisis parmi les conservateurs, les régulateurs d'acidité, les antimousses, les arômes et les colorants en une quantité de 0 à 10 g, et
e) de l'eau jusqu'à 100 ml,
la composition comprenant une quantité d'alcools de sucre de moins de 10 g pour 100 ml de la composition.

2. Composition selon la revendication 1, dans laquelle la composition présente une viscosité dans la plage allant de 50 mPas à 30 Pas à une température de 20 °C.

3. Composition selon l'une quelconque des revendications précédentes, contenant un conservateur approprié.

4. Composition selon la revendication 3, dans laquelle le conservateur est contenu en une quantité de 0,01 g à 0,5 g pour 100 ml de la composition.

5. Composition selon l'une quelconque des revendications précédentes, dans laquelle la composition présente un pH dans la plage allant de 2,0 à 6,0.

6. Composition selon l'une quelconque des revendications précédentes, dans laquelle la composition présente une teneur en eau d'au moins 70 % en poids.

7. Composition selon l'une quelconque des revendications précédentes, dans laquelle la composition est un jus antitussif ou un sirop antitussif.

8. Utilisation d'une composition selon l'une quelconque des revendications précédentes pour l'utilisation pour le traitement sécrétoloire lors de bronchopneumopathies aiguës ou chroniques.
